Europäisches Patentamt

European Patent Office

Office européen des brevets

(11) Publication number: **0 363 703 B1**

# EUROPEAN PATENT SPECIFICATION

(45) Date of publication of patent specification: **22.02.95**  (51) Int. Cl.⁶: **C12N 5/22**, C12P 21/08, A61K 39/395

(21) Application number: **89117479.9**

(22) Date of filing: **21.09.89**

(54) Method of producing human-human hybridomas, the production of monoclonal and polyclonal antibodies therefrom, and therapeutic use thereof.

(30) Priority: **23.09.88 US 248082**

(43) Date of publication of application:
**18.04.90 Bulletin 90/16**

(45) Publication of the grant of the patent:
**22.02.95 Bulletin 95/08**

(84) Designated Contracting States:
**AT BE CH DE ES FR GB GR IT LI LU NL SE**

(56) References cited:
**EP-A- 0 057 107
EP-A- 0 124 717
EP-A- 0 202 642**

**IMMUNOLOGY SERIES, vol. 30, Human Hybridomas, Diagnostic & Therapeutic Applications, 1987, Marcel Dekker, pages 23-38.**

**JOURNAL OF IMMUNOLOGICAL METHODS, vol. 100, 1987, pages 5-40, Elsevier Science Publishers BV.; K. James et al.: Human monoclonal antibody production current status and future prospects**

(73) Proprietor: **Alonso, Kenneth
2921 Margaret Mitchell Court, NM
Atlanta
Georgia (US)**

(72) Inventor: **Alonso, Kenneth
2921 Margaret Mitchell Court, NM
Atlanta
Georgia (US)**

(74) Representative: **Patentanwälte Grünecker,
Kinkeldey, Stockmair & Partner
Maximilianstrasse 58
D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

BACKGROUND OF THE INVENTION

This invention relates to a method of producing human-human hybridomas, and to the production of monoclonal antibodies from the hybridomas. This invention also relates to the use of the monoclonal antibodies for preparing a medicament for the treatment of human disorders.

The first hybrid-myeloma, or hybridoma, resulted from the fusion of mouse myeloma cells with lymphocytes from the spleen of mice immunized with a particular antigen. The fused or hybridoma cell expressed both the specific-antibody production of the parent spleen cell and the immortal character of the myeloma cells. It was soon recognized that individual hybrid cells could be cloned and that each clone produced large amounts of identical antibodies to a single antigenic determinent. Individual clones can be maintained indefinitely and at any time samples can be grown in culture or injected into animals for a large scale production of monoclonal antibody. This technology was soon extended to production of antibodies from mouse myeloma cells.

The therapeutic uses of such antibodies in man seemed obvious. It has been found, however, that use of mouse- and rat-based antibodies are only transiently useful in therapy of certain malignancies, especially leukemia and lymphoma. For example, antibody trials in human T-cell leukemia and lymphoma have been attempted using mouse- or rat-based monoclonal antibodies with T-cell specificity. Using antibody to Leu-1, a transient fall in the leukemic cell count in the peripheral blood of patients with circulating malignant T cells has been reported. While the mouse- or rat-based antibodies were effective in causing sharp drops in white blood cell counts, the therapeutic effects were brief, lasting only hours. A more sustained response was achieved when monoclonal anti-idiotype antibody was administered by pulse therapy. See Miller et al., Response of Cutaneous T-cell Lymphoma to Therapy with Monoclonal Anti-idiotype Antibody, New Eng. J. Med. 306: 517-522 (1982).

Similar transient reductions in tumor have been recorded at the National Cancer Institute in patients with chronic lymphocytic leukemia, with cutaneous T-cell lymphoma and with common acute leukemia. Antibody bound to leukemic cells in the marrow and skin has been demonstrated by immunoperoxidase and flow cytometry analysis. Patients with cutaneous T-cell lymphoma have shown minor responses with antibody therapy directed at T-cell antigen.

Results in malignant melanoma and solid tumors have varied widely depending upon tumor size, and whether the antibody fixes, complements or initiates antibody-dependent or cell-mediated toxicity.

The mechanisms for the therapeutic effects of monoclonal antibody are not clear. Complement-mediated cytotoxicity, antibody-dependent, cell-mediated toxicity, and direct antiproliferative effects have been postulated. It is hypothesized that the reticuloendothelial system plays a major role in antibody effect because the reticuloendothelial system removes coated circulating cells. Further, lymphocyte agglutination, or trapping, in the lungs after antibody treatment of leukemia has been described. Conversely, the lack of effect of antibody therapy on bone marrow leukemic cells has also been noted.

Antigenic modulation where the antigen disappears from the cell surface by shedding or internalization has been described within hours after the administration of antibody. The modulation is temporary with return of the antigen following discontinuation of administration. It does not occur with all antigens after exposure to monoclonal antibody. Antigenic modulation has been observed more with the antigens on hematopoietic cells than with those on solid tumors. Modulation may be a mechanism by which an immunoconjugated molecule could gain entrance into the cell. Antigen shedding, however, may lead to increased reticuloendothelial system uptake of antibody complexes.

In addition to the transient nature of the therapeutic effect obtained when mouse- and rat-based antibodies are administered to humans, it has also been found that these antibodies can cause immune reactions that interfere with the desired therapeutic effect. The use of other than human cells for hybridoma formation is associated with significant generation of an anti-species immune response by the patient. In one study, antibody was produced in a variety of different species in order to permit continued administration of the antibody while evading host response to any individual "xenograft" in the patients treated. Nevertheless, large numbers of patients rapidly mounted a response to the animal-derived protein. It has also been noted that the use of mouse-based antibodies is associated with increased uptake and clearing by the reticuloendothelial system.

Toxicity and side effects associated with intravenous infusions of mouse-based antibodies has been well described in one hundred and two patients with ten different malignancies. R.O. Dillman, et al., "Toxicity and side effects associated with intravenous infusions of murine monoclonal antibodies," J. Bio. Res. Med. 1986; 5:73-84. The principal side effect is that of bronchospasm which is controllable with low infusion rates (20 mg/hr). Urticaria is common as well. A large percentage of patients do manifest anti-mouse antibodies following infusion and repeated challenge to the immune system may lead to the development of anemia. These immune reactions remain the chief limitations to widespread use of non-human based monoclonal antibody for imaging or therapy.

A more specific approach in the use of monoclonal antibody was described by Miller et al. in "Treatment of B-cell lymphoma with monoclonal anti-idiotype antibody", N. Eng. J. Med. 306: 517-522 (1982). One patient with an IgM producing B-cell lymphoma resistant to cytotoxic drugs and to interferon was treated with an anti-idiotype antibody twice weekly for four weeks. Systemic signs disappeared after four low dose infusions. With increase of the dose to 75 mg during the fifth treatment the tumors began to regress. After the eighth treatment, the patient was in complete remission and has remained so for four years. In ten subsequent patients with B-cell lymphoma treated at Stanford, five patients had partial responses. K. Meeler, et al., "A clinical trial of anti-idiotype therapy for B-cell malignancy," Blood; 65:1345-1363 (1985). A direct antiproliferative or growth regulating effect through non-neoplastic cells has been postulated.

Therefore, there is a need for species specific and tumor specific antibodies to treat disease. In particular, there is a special need for monoclonal antibodies that can be injected directly into a patient to bolster the patient's own antibody response by augmenting and stimulating the patient's natural immune mechanism and by finding and attacking specific tumor cells. Ideally, the antibodies should provide more than a mere transient therapeutic effect. In addition, the antibodies should not cause immune reactions that interfere with the desired therapeutic effect. It is anticipated that antiidiotype antibodies produced from human hybridomas will cause regression of the targeted malignancies.

## SUMMARY OF THE INVENTION

This invention aids in fulfilling these needs in the art by providing a method for the production of human-human hybridomas.
The method comprises the steps of:

(a) providing a suspension comprising cells from a human tumor and a suspension comprising human spleen cells;

(b) sensitizing the human spleen cells in the suspension by mixing the cells with human tumor cells in the presence of a spleen cell stimulating agent;

(c) fusing the human spleen cells and the human tumor cells with B-lymphocytes from a cell line distinct from the previously sensitized spleen cells;

(d) screening the resultant fused cells to select at least one hybridoma that produces antibodies reactive with a specific human tumor antigen; and

(e) cloning the selected hybridoma.

In addition, this invention provides a method of producing monoclonal antibodies by culturing a hybridoma of the invention in a culture medium and recovering the antibodies from the culture medium. Monoclonal antibodies of the invention include, for example, monoclonal antibodies to adenocarcinoma of the colon, adenocarcinoma of the lung, adenocarcinoma of the breast, mucoepidermoic carcinoma, hepatocellular carcinoma, leiomysarcoma, melanoma, neurofibrosarcoma, squamous carcinoma of the tongue and of the anus, adenocarcinoma of the pancreas, lymphoblast (acute leukemia), Mycosis Fungoides, oat cell carcinoma, adenocarcinoma of the prostate, squamous carcinoma of the esophagus, adenocarcinoma of the stomach, adenocarcinoma of the biliary tract adenocarcinoma of the ovary (mucinous and serous lymphoblast (lymphoma), glioblastoma, and alveolar cell carcinoma of the lung.

Further this invention provides a method for producing a continuous cell line that produces monoclonal antibodies to a specific cancer. The method comprises the steps of:

a) sensitizing human spleen cells in suspension by mixing the cells with a spleen cell stimulating agent in the presence of human tumor cells;

(b) fusing the human spleen cells and the human tumor cells with human fetal marrow or lymphoblastic cells to produce hybridomas;

(c) selecting from among said hybridomas a hybridoma that produces antibodies reactive with only one human tumor antigen; and

(d) clonally expanding said selected hybridoma into a cell line.

In summary, the method for making human-human hybridomas, herein described can produce large amounts of cost effective specific antiidiotype antibody useful in the treatment of solid tumors as well as leukemia and lymphoma. Moreover, the antibodies of the claimed invention, which are both monoclonal and polyclonal, meet a longfelt need because they do not cause the immune reactions produced by mono and polyclonal antibodies from inter-species hybridomas.

The antibodies of the present invention also can be used in the diagnosis of cancer (analogous to the CEA); in diagnostic imaging by attaching the metal Gadolinium to facilitate its use in MRI or Technetium or Indium to facilitate its use in nuclear medicine scanning; as a carrier for other compounds to localize tumors and in radio therapy of tumors as described in Alonso et al., "Radiolabelled Monoclonal Antibody to Pancreas Carcinoma; Preliminary Evaluation for Adjunctive Use In Treatment", Southern Medical Journal, Vol. 79 (Supp. 2):27 (1986), specifically incorporated herein by reference.

DETAILED DESCRIPTION OF THE INVENTION

Monoclonal antibody is produced in a human-human system in serum-free culture as described by Reading in "Theory and Methods for Immunization in Culture and Monoclonal Antibody Production", J. Immunol. Methods 53:261-291 (1982), specifically incorporated herein by reference, and characterized by SDS-PAGE electophoresis and Western Blot immunoavidity studies. Fresh tumor is minced, meshed, and a single cell suspension prepared. Tumor cells are washed with Dulbecco's Minimum Eagle's Medium (DMEM) (obtained from GIBCO) and are used to sensitize human spleen cells. Tumor cells in a concentration of $10^6$/ml in DMEM F12 are placed with spleen cells in a concentration of $10^6$/ml and incubated in a $CO_2$ enriched atmosphere at 37°C.

Sensitizing the spleen cells means that foreign (i.e., cells not from the same donor as the spleen) surface cell antigens are placed in contact with spleen cells. These cells identify the antigens as foreign and initiate the immune response that characterizes the nature of their foreignness.

After 4 days incubation, the mixed cultures of both cells are harvested, washed with DMEM and are ready for fusing.

Sensitized spleen and tumor cells in a concentration of $10^6$ cells/ml are combined with a fusion line at a concentration of $10^6$ cells/ml in 50% polyethylene glycol (Sigma, St. Louis, MO) in DMEM.

Three distinct cell lines have been used for fusion; they are a fetal marrow line, a lymphoblastoid line and a plasma cell line from myeloma.

The sensitization response to surface antigen is non-specific and thus the resulting clones must be examined to find one with the desired antibody. Therefore, the cell suspension is then plated in DMEM F12 into a 96 well tissue culture plate and incubated in a $CO_2$ enriched atmosphere at 37°C. Those clones with antibody production as demonstrated by color change with ELISA and showing no cross-reactivity with a variety of cell types are expanded for antibody production in tissue flasks. Antibody recovered is precipitated in 50% ammonium sulfate in ice and dialyzed for 48 hours against 0.15M sodium chloride with three changes of buffer. The material is recovered by chromatography through a sepharose column and fractions collected. Ultrafiltration (BioRad, Richmond, CA) may concentrate the specimen further. An indirect two-stage immunoperoxidase procedure is employed for histochemical analysis.

Interleukin-2, which can be used to sensitize the tumor and spleen cells employed in the hybridoma, is raised from human lymphocytes and isolated employing the method of Gallo described in Proc. Nat. Acad. Sci. USA: 77: 6134-38(1980), specifically incorporated herein by reference. Briefly, buffy coats obtained by leucophoresis from HIV, HBV negative donors are supplied by the American Red Cross (Atlanta, GA). Buffy coats are segregated according to blood type and cells separated on Ficol-Hypaque (Sigma, St. Louis, MO). These are washed in Dulbecco's modified Eagle's medium (DMEM) (Gibco, New York) and stimulated with 1% v/v Phytohemagglutinin-P (Sigma, St. Louis, MO) in DMEM. After 4 days incubation in a $CO_2$ enriched atmosphere at 37°C, the supernatant is recovered, and non-interleukin-2 protein precipitated with 55% ammonium sulfate (Sigma, St. Louis, MO). The supernatant is dialyzed, refrigerated, in TRIS buffer (pH8) (Sigma, St. Louis, MO) with renewal of the dialysis bath. Dialysate is then passed through a Sepharose column (Pharmacia, Piscataway, NJ) and eluted with 0.02m sodium chloride (pH8). Fractions with the highest absorbance are then pooled for a second chromatography step with Sephadex (Pharmacia, Piscataway, NJ) and fractions collected. One unit is defined as that which quintuples interleukin-2 dependent, PHA independent cells with four days of exposure.

All other agents employed are obtained from licensed distributors of pharmaceutical products.

Eight patients have presently been treated in an anti-idiotypic antibody study. Bone marrow purging with antibody was completed for one autologous bone marrow transplant patient. Antibody infusion of up to 800 mg. is tolerated without difficulty and binding of anti-idiotypic antibody to tumor tissue is striking in biopsy materials obtained as late as 48 hours after infusion. In vivo binding is compatible with binding noted between the biopsy material and antibody ex vivo prior to infusion. Nonspecific absorption by red blood cells is not seen. Nonspecific binding is not evident in biopsy materials obtained after infusion and is compatible with findings noted ex vivo prior to infusion. Tumor regression has been noted during infusions. Maximal shrinkage is noted at 4 days and some regrowth by 7 days. Follow up over 4 weeks showed no anti-human antibody formation, alteration in renal or hepatic function, alteration in thyroid or adrenal function, or circulating antigen-antibody complexes. Complement-activated clearing of immune complexes increased following infusion.

The preparation and characterization of the hybridoma and the resultant anti-idiotypic antibodies will be better understood by reference to the following examples.

EXAMPLE 1 - Production of a Human Tumor-Human Lymphocyte Hybridoma

All procedures described below meet Good Laboratory Practice Standards.

Preparation of Tumor Cell Suspension: Tumor is received fresh in saline or in (other) tissue culture/viral transport medium. The specimen is minced in a laminar flow hood utilizing a sterile scalpel and forceps. The minced tissue is then placed on a 100 mesh screen and grated. The processed material is washed from the grid with sterile DMEM medium into a sterile petri dish although any tissue culture medium may be used. Tissue fragments remaining in the grid are removed with forceps and placed in a glass tissue homogenizer for further separation. Tissue is ground by the vertical (up and down) movement of the central tube in conjunction with the rotation of that central tube. DMEM medium is used to wash the holding receptacle of the tissue homogenizer and, with the central tube placed, leaving minimal clearance between the receiving vessel and the central tube, the cellular material is decanted into the sterile petri dish. This pooled material is then aspirated into a plastic syringe and forced through a 21 ga. needle into a second dish. That material is then aspirated and passed as above through a 23 ga. needle. The process is repeated a third time with the material passed through a 25 ga. needle. The disrupted specimen, now a single cell suspension, is then placed in a graduated polycarbonate conical tube with a Pasteur pipette. The cells are then washed with an equal volume of DMEM medium and centrifuged at 200xg for ten minutes. The supernatant is discarded, the cellular material resuspended with a Pasteur pipette, and the material washed again with an equal volume of DMEM medium. This is centrifuged a second time at 200xg for ten minutes at room temperature.

The cell suspension is counted on a hemocytometer. A 1:100 dilution is made of the cell suspension utilizing a Sahli pipette. The diluting fluid is comprised of a 1:1 mixture of 0.2% trypan blue with saline. The diluted fluid is mixed with the cell suspension by hand rotation and, after five minutes exposure at room temperature, pipetted onto the counting chamber of a hemocytometer (in duplicate). The number of cells in the nine large squares are counted. Those which do not take up the dye are noted separately. The number of viable cells is determined as follows:

cells without dye/total cell count x 100% = % viable.

That material placed on the hemocytometer is then reaspirated, centrifuged, and a cell block prepared for histologic examination.

The cell suspension is diluted to a volume containing $10^7$ cells/ml viable tumor cells utilizing Dulbecco's minimum Eagle's medium (DMEM). Graduated polycarbonate tubes (conical bottom) are prepared by adding 4 ml Ficoll-Hypaque. The cell suspension (no more than 10 ml per tube) is then layered onto the Ficoll-Hypaque by holding the polycarbonate tube at an angle and using a Pasteur pipette to transfer the suspension along the walls of the tube, slowly, not disrupting the clear viscid medium at the tube base. Centrifuge at 2000 xg for 20 minutes at room temperature. Collect all the fluid above the pellet. This contains the viable cells and is free of red cell contamination. Add 10 ml DMEM and centrifuge at 300 xg for ten minutes. Decant. Wash the pellet twice with DMEM and centrifuge each time at 250xg for 10 min.

The cell suspension is then placed in a tissue flask (which flask may be coated with epithelial cell matrix material or adhesive protein from marine mussel to facilitate growth of the cells) and 10 ml DMEM F12 added.

Insulin, progesterone, or other growth stimulants are added to the flask containing the tumor cell suspension. Transferrin is supplied to facilitate iron transport to the cells. It is preferred that no serum and no antibiotics be added in order to avoid viral contamination and to facilitate clean up of the antibody prior to use. Serum-free and antibiotic-free growth is more difficult to maintain; therefore, if needed human AB rh negative serum and Gentamicin may be used. The flask is bagged with a $CO_2$ generator and placed in a 37°C incubator.

Growth is examined at the end of seven days. Medium is changed earlier if the indicator dye in the medium changes color. Cells are removed from the flask mechanically, washed in DMEM, and centrifuged at 250xg for ten minutes (twice).

The cell suspension is reconstituted to $10^6$ cells/ml and 1 ml is taken for cryopreservation. The remainder of the specimen is available for immunization and study.

CRYOPRESERVATION: A suspension of $10^6$ cells/ml in DMEM is added to labelled and dated freezing tubes. AB rh negative human serum is also added to bring to a concentration of 10%, although HBV and HIV free human serum may be used, autologous serum is preferred. Cool in ice for 15 minutes. Add DMSO (Dimethyl Sulfoxide) to a final concentration of 10% while gently shaking the cell suspension. Invert several times for thorough mixing. Insert into the vapor phase of liquid nitrogen in a liquid nitrogen container. The freezing tube must remain for a minimum of two hours in the vapor phase but may remain in that environment for a longer period. The container is then removed from the apparatus, placed in a cardboard cane, and stored in the liquid nitrogen.

These cells may be thawed quickly when needed for use in a 37°C water bath without agitation to the point of transition to the liquid phase. Then quickly transfer the cells into a polycarbonate conical centrifuge tube and dilute tenfold with DMEM with 10% AB rh negative human serum. Wash twice by centrifugation at 200xg for ten minutes. Resuspend and place in flask.

SENSITIZATION: Spleen cells obtained from a human fetus or adult are employed to sensitize the tumor cells prior to fusion. The fresh fetal or adult spleen is placed in a sterile setting and the capsule is teased with sterile instruments. The splenic pulp is then prepared as described in the preparation of tumor cell suspension, supra. The cells are maintained cryopreserved and must be thawed and replicated prior to sensitization. Frozen lines are stable when thawed. Human spleen cells are stimulated prior to fusion with (1) transfer factor, (2) common mitogens that stimulate blast transfer such as PHA or Con A, or (3) specific lymphokine which does not depress immune response such as IL-2. All have been used and are equally effective. 2-mercaptoethanol may be added to the culture medium to enhance spleen cell growth.

Tumor cells are prepared in a concentration of $10^6$/ml in DMEM F12. Spleen cells are washed three times in DMEM and centrifuged each time at 200xg for ten minutes to remove serum traces. Spleen cells are then prepared at a concentration of $10^6$ cells/ml. Interleukin-2(ly)10 units/ml is added to the suspension. Serial two fold dilutions are made with DMEM F12 to a dilution of 1:16. 0.1 ml tumor cells and 0.1 ml spleen cells are mixed together to form the zero dilution. Dilutions are plated in a 24 well plate and medium added to bring the total volume in each well to 1 ml. After preparing all the cultures, secure the lid, bag, and place with a $CO_2$ generator in a 37°C incubator. Refeed daily with 0.05 ml DMEM F12. Rebag and add generator. After 4 days incubation, cultures of both cells are harvested by scraping each well with a policeman and transfering the contents with a Pasteur pipette to polycarbonate tubes and pelleted by centrifuging at 200xg for ten minutes. The cells are washed with DMEM and are ready for fusing.

SCREENING PLATES: At the time tumor cells are prepared for sensitization, tumor cells are also dispensed to each well of a 96 well microplate (0.05 ml suspension to each well). Cells are allowed to dry and are fixed with 70% alcohol. Prior to using after fusion experiments to screen for antibody formation, the wells are washed with saline and are flicked dry. The plates are blotted.

As many plates as can be prepared with the working suspension should be prepared and labelled for later use. Redundant plates may be used for cross-reactivity screening.

FUSION: Three distinct cell lines have been used for fusion: they are a fetal marrow line, a lymphoblastoid line and a plasma cell line from myeloma. The fetal marrow line is distinct from that of the fetal spleen line used to sensitize the tumor cells. All fusion lines are prepared at $10^6$ cells/ml and screened for antibody production. Positive wells are stimulated with IL-2 or common mitogens or transfer factor and cloned by limiting dilution (see p. 19, infra). Single clones producing only one class of immunoglobulin are selected. This is determined by immunoperoxidase staining as described at p. 18, infra. An IgG line from fetal marrow (BG-231), an IgM line from lymphoblastoid tissue (BM-95) and an IgA line from myeloma (BA-160) have been successfully used. These three fusion lines have been deposited in the American Type Culture Collection on September 16, 1988 under ATCC Nos. CRL 9835, CRL 9832 and CRL 9834 respectively. All lines are maintained cryopreserved and must be thawed and replicated prior to fusion. All lines are stable when thawed.

The line employed for fusion is grown to a density of $10^5$ cells/ml. Following washing the DMEM, viability is determined with trypan blue exclusion and exceeds 95%. Wash three times with DMEM.

Sensitized tumor and spleen cells in a concentration of $10^6$ cells/ml are mixed with a fusion line at a concentration of $10^6$ cells/ml and are placed in a polycarbonate centrifuge tube and centrifuged to 400xg for 10 minutes. Remove supernate from the pellet and maintain in a 37°C water bath. Using a 1 ml pipette add 1 ml warm 50% polyethylene glycol (PEG) to the pellet over a one minute period. Gently stir the cell pellet with the pipette tip as the polyethylene glycol is being added. Continue to stir for a second minute until the cell suspension looks like a homogeneous clump of cells. With the same pipette, stir in 1 ml DMEM which has been warmed to 37°C. This should dilute the PEG without lysing the cells. Finally, with a 10 ml pipette stir in 7 ml of DMEM over 2-3 minutes. Do not pipette. Then centrifuge the specimen at 400xg for 10 minutes to remove the supernate. Add 30 ml DMEM by aiming the pipette tip at the cell pellet and stirring to obtain a suspension of cell clumps. Plate 0.1 ml of this suspension into each well of three 96 well tissue culture plates and bag with a $CO_2$ generator and place in an incubator at 37°C.

On the day after the fusion, add 0.1 ml hypoxanthine-aminopterin-thymidine (HAT) medium to each well. On days 2, 3, 5, 8, and 11 respectively, aspirate off half the medium from each well and add 0.1 ml HAT to each well after aspiration. Rebag and add $CO_2$ generator each time before placing in incubator.

At day 14 begin replacing medium with HT every to 3-4 days. By day 24 the cells are transferred to DMEM F12 alone. Alternatively, one may omit HAT and HT and use DMEM F12 alone from day 1. Fused cells are stable in culture plates for up to 6 weeks.

INITIAL SCREENING: Between two and four weeks after cell fusion and prior to refeeding, the supernate is harvested using individual pipettes for each culture plate well. These are to be tested at a dilution of 1:10 (one part supernate to nine parts saline).

Retrieve the antigen coated plates prepared earlier. Flick the contents into a sink and pat dry on a clean towel. With a gentle stream fill the plate wells with Phosphate Buffered Saline (PBS, pH 7.4). It is necessary to check that no air bubbles are trapped in any of the wells. Flick, dry, and refill with PBS twice. Add 0.05 ml of each hybridoma supernate to the antigen precoated wells. Add an additional 0.05 ml PBS to each well. Incubate at room temperature at least one hour.

Antihuman globulin conjugated to peroxidase is diluted 1:100 with PBS. Flick the contents of the supernate incubated plates into a sink and pat dry. Wash each well with a gentle stream of distilled water. Check that no air bubbles are trapped in any well. Wash until no air bubbles are trapped. Flick, dry, and refill with water twice. After the last wash, add 0.10 ml diluted anti-human globulin peroxidase conjugate to each well and incubate the plates at room temperature (18-20°C) for at least one hour but not more than two hours. Citrate buffer containing 1% urea peroxide is prepared and the chromophore, o-phenylene diamine, is added and dissolved. Flick and dry the plates. The wells are washed with distilled water three times. Check that no air bubbles are trapped in any well. Wash until no air bubbles are trapped. Following the last wash, add 0.10 ml substrate cromophore solution in citrate buffer to each well. Incubate at room temperature at least 20 minutes but not longer than 30 minutes as the color change beyond that point is independent of globulin content or substrate/chromophore availability.

Positive wells will develop a yellow amber color; negative wells will retain a clear to pale yellow hue.

CLONING BY LIMITING DILUTION: Those cells making antibodies of interest are transferred into 1 ml tissue culture wells of a 24 well plate. Place 0.5 ml HT or DMEM F12 medium into each well. Use DMEM F12 as a base 1 medium. Resuspend the contents of each antibody producing master plate well with a pipette and transfer the entire suspension to the transfer well. Resuspend this mixture and then add 5 drops of this suspension to the original master plate well. This creates duplicate cultures. After 2-3 days, refeed with 0.5 ml HT or DMEM F12 medium.

Two days later, remove supernate and add fresh HT or DMEM F12 medium. When growth is nearly confluent, rescreen for antibody activity as above. If the culture continues to produce the antibody of interest, the cells are split. One split is for cloning. The second is expanded for freezing and storage. The mixture to be frozen is placed into a flask containing 5 ml DMEM with 10% AB rh negative human serum, bagged, placed with a $CO_2$ generator, and incubated at 37°C. The freezing procedure is described above.

Remove the cells of interest from the 1 ml cultures and determine the concentration of viable cells with trypan blue. This has been described earlier. Dilute a sample of the culture to be cloned so that 230 live hybrid cells are suspended in DMEM F12 with growth factors (insulin, progesterone, vitamin C, transferrin). Plate 36 wells of a 96 well plate with a 0.1 ml of this mixture. To the remaining 1.0 ml of cell suspension add another 4.0 ml of DMEM F12 and replate another 36 wells. To the remaining cell suspension add 1.4 ml DMEM F12 and dispense the remaining mixture, again in 0.1 ml aliquots.

On day 5 and again at day 12, feed the plate by adding 0.05 ml DMEM F12. By day 14 the clones should be large enough to test. One of the three dilutions plated should yield some wells with no growth (if an average of 1 cell/well is plated, 37% of the wells should have no growth). Wells that appear to be monoclonal should be tested again for antibody activity.

Transfer 6 positive clones into separate 1 ml culture wells of a 24 well plate and add HT medium (in DMEM as base) or DMEM F12 alone as described earlier. Test the supernates for antibody as described above. If continued positivity is demonstrated, transfer the positive clones into 5 ml flasks and further expand the cultures with DMEM F12. Freeze a portion of the clone as described earlier.

ANTIBODY PRODUCTION: Positive clones are placed in stationary flasks (which may be coated with epithelial cell matrix coating or adhesive protein from marine mussel) and DMEM F12 added. The cells are allowed to overgrow. The flasks are maintained bagged, in a 37°C incubator, with a $CO_2$ generator placed. Weekly the supernate is removed and the flask replicated. The supernate is retained and multiple supernates pooled for purification. The supernate is stable at room temperature or at 37°C or in the refrigerator for extended periods. The replicated flasks permit maintenance of a master working cell bank to insure that all harvests are from the same growth phase. For increasing scale of production positive clones are removed from stationary flasks and placed in spinner flasks with DMEM F12 added. The flasks are maintained in a 37°C water bath with atmosphere monitored to maintain 5-7% $CO_2$/ 93-95% air. The flasks are stirred continuously at 15 rpm to minimize shear damage to cells. Supernate may be removed continuously with addition of DMEM F12 or removed daily with DMEM F12 added.

PURIFICATION: Immunoglobulins are precipitated in 40% saturated ammonium sulfate (pH 7.4): The supernate is centrifuged at 2,000xg for 30 minutes. To the immunoglobulin collection the ammonium sulfate is added slowly with stirring until 40% saturation is reached. The material is then held for 16 hours in ice. The precipitate is resuspended and centrifuged at 2,000xg for 30 minutes. The precipitate is then dissolved in a minimal amount of water and dialyzed for 48 hours against 200 volumes of 0.15M NaCl (pH 7.4) with three changes of buffer. The dialysis membrane is pretreated with buffer prior to introduction of the material to be dialyzed.

The dialyzed material is recovered, and added to a column of DEAE-Sepharose (25x50mm). The material is eluted with the stepwise addition of 0.05M NaCl (pH 7.4) at low pressure. Five ml fractions are collected. Two hundred volumes of eluent are employed for each volume of dialysate. The fractions are examined at 280 nm and those with the highest O.D. are retained. A UV spectrum is then obtained (190-340nm) to examine purity. Transferrin alone is the contaminant noted in the dialyzed material and this is removed by gel chromatography. The specimen may be concentrated further by ultrafiltration. Centrifuge aliquots at 2,000xg for 20 minutes in a membrane separated tube. Recover upper layer.

CHARACTERIZATION: Cellular samples are reconstituted in saline to a protein concentration of 1 mg/ml. Refractometry comparison to a known protein standard is utilized to approximate the protein concentration of the antigen. The material is then solubilized by the addition of Seprasol with 2% agarose in a 1:1 ratio of antigen preparation to solubilization solution. Begin with at least 0.5 ml antigen solution. Heat the mixed sample to 60°C for five minutes and then draw into 0.2 ml pipettes to cool prior to addition to the gels. Prestained molecular weight standards are diluted 1:2 in Seprasol with 2% agarose, heated at 60°C for three minutes, and drawn into 0.2 ml pipettes to cool prior to addition to the gels. The standards are allowed to cool in the gels.

Molecular weight standards are ejected from the pipettes as 1.5 mm diameter rods and cut to 1 cm lengths. Each Sepragel receives two 1 cm molecular weight standards which are placed 1 cm from the edges at the tops of the resolving gel. A 1.5 mm deep layer of molten 1% agarose in the SDS running buffer is added over the rods to affix them to the gel. When the plug has solidified, the antigen sample is overlayed into the well above the gel and overlayed with the running buffer agarose and allowed to solidify at room temperature.

When all samples have been applied and cooled, the gel is placed into the electrophoresis tank and electrophoresed at 200v (constant voltage) in SDS running buffer at ambient temperature until the tracking dye (bromphenol blue) has migrated out of the cassette. The cassettes are then removed from the tank, opened, and the gels transferred to plastic tubs containing 25mM TRIS, 192mM glycine in 10% methanol and equilibrated for 30 minutes at room temperature.

The electrophoresed materials are transferred to Immobilon PVDF membranes by placing onto membranes in the electrophoresis tank and using the cooling coil, transferring in an anodal direction using Tris glycine buffer in methanol at 150 volts constant voltage at room temperature for 4 hours. The membranes are then removed and rinsed in PBS with 0.05% Tween 20 and set out on filter paper to dry. The membranes are then cut into 1 cm strips and labelled with a two digit code indicating antigen and antibody employed.

Strips are then wet by rapid passage through 100% methanol and rinsed in PBS with 0.5% Tween 20. The strips are then placed in 13x100 screw top test tubes with 10 ml 5% bovine serum albumin in PBS with 0.5% Tween 20 and rocked at 37°C for 20 minutes. After rocking, the solution is decanted and replaced with primary antibody solution. The antibody solution is prepared at a concentration of 1 mg/ml and diluted 1:10 and 1:100 in PBS with 0.05% Tween 20. The solution is incubated with the strips for 1 hour at room temperature (control strips are incubated without primary antibodies). The strips are then decanted and rinsed with PBS and 0.5% Tween 20 for 10 minutes at room temperature.

The strips are incubated in 3 mls each of a 1:2000 dilution of anti-human/alkaline phosphatase at room temperature for one hour. Strips are rinsed for 20 minutes, twice, in PBS with 0.5% Tween 20. 10 mls of each solution is required. Strips are soaked in TRIS (100mM, pH 8.8), NaCl (100mM), $MgCl_2$ (5mM) buffer for 10 minutes. When adequately developed, the strips are rinsed in water and allowed to dry.

Nitroblue tetrazolium (NBT) salts are dissolved in 70% N,N-dimethyl formalide (NNDMF) in water to a concentration of 75 mg/ml 5 bromo-4-chloro-3-indilyl phosphate (BCIP) is prepared by dilution of 50 mg in 100% NNDMF. These are made fresh with each application.

0.07 ml NBT is added to 25 ml TRIS, NaCl, MgCl$_2$ buffer, mixed well, and combined with 0.01 ml BCIP and added to the vessels containing the strips. At least 3 ml per test tube is required. Read when adequately developed.

Migration allows estimation of molecular weight (standard migrates in first dimension); reaction characterizes immunoglobulin type; immunoreactivity demonstrated in second dimensional transfer.

BINDING: Cell lines of interest include that of the tumor from which the antigen has been prepared as well as a cell panel which includes tumors of glial, breast, lung, stomach, liver, colon, ovary, and prostate origin. Cell lines are prepared to 10$^4$/ml and reacted with antibody dilution determined above. This is done by adding 0.2 ml antibody to 0.2 ml cell line in DMEM. Following 30 min. incubation at 37°C the cells are pelleted by centrifugation after washing with DMEM (400xg, 5 min). A 1:2000 dilution of anti-human/peroxidase secondary antibody is then added to the pellet reconstituted in DMEM and incubated for 30 mins. at 37°C. The color is developed after washing with DMEM and pelleting by centrifugation (400xg, 5 min) by adding citrate buffer with urea peroxide and incubating for 15 mins. The cell preparation is then placed in a hemocytometer and color development evaluated. The percentage of those cells with demonstrable color development are determined by counting.

FETAL CELL SCREENING: Frozen section preparations of fetal tissues are prepared. Brain, thymus, lung, heart, liver, spleen, pancreas, adrenal, stomach, colon, ovary, prostate, bone marrow, and lymph node are examined. The slides are first washed with PBS for 5 minutes in the cold and primary antibody is placed for 5 minutes in the cold. The slide is then washed again with PBS. The secondary antibody with peroxidase is then applied for 5 minutes, washed, and incubated with citrate buffer with urea peroxide. Control slides include those treated with bovine serum albumin as well as those pretreated with peroxidase. Color development is evaluated microscopically and scored as to intensity. The slides are counterstained with Harris Hematoxylin.

NUCLEOTIDE CONTAMINATION: Ten ng known DNA are added to 1 ml crude antibody preparation prior to dialysis. This spiked preparation is then carried through parallel purification steps and electrophoresed as noted above.

Biotinylated probe is added to the test strip, rinsed with buffer, and color developed. One strip is spiked with 100 ng known DNA. Absence of color in the purified strip indicates the level of reduction obtained.

The Nick Translation kit (Enzo Biochem) is employed to screen the material for polynucleotide contamination. The DNAse solution supplied is diluted 1:500 with a 10mM TRIS HCl buffer containing 1 mg/ml nuclease free bovine serum albumin (BSA). Two reaction vials are prepared. One contains a known DNA control provided in the kit. The second contains the antibody preparation being examined following purification. Into each vial is placed the TRIS hydrochloride buffer with BSA, the nucleotide solution provided in the kit, biotinylated deoxy UDP, DNA polymerase, and the diluted DNAse solution. These are incubated for 2 hours at 14°C. At the end of two hours the reaction is stopped with 0.2M EDTA. The material is then placed at 65°C for 10 minutes and is ready for incorporation analysis.

Denaturation of DNA is undertaken by rapidly boiling and cooling the mixture. This is added to Denhardt's solution and to a solution of 0.1% SDS with 50% formamide, 3.6M NaCl, 0.2M Na$_2$PO$_4$, and 0.2M EDTA. Filters provided in the kit are then placed in the formamide solution for ten minutes to prepare them for inoculation. After pretreatment the Denhardt's mixture is added and the filters are bagged and incubated 4-6 hours at 42°C. This hybridization solution is then added again at a 0.2 ml/cm$^3$ concentration, the filters bagged, and incubated for 16 hours at 42°C. With completion of the incubation the filters are washed in the sodium chloride/sodium phosphate/formamide/SDS buffer one at room temperature for 15 minutes and then twice more at 60°C for 15 minutes. The filters are washed a final time in sodium chloride/sodium phosphate at room temperature and are now ready for detection with avidin-peroxidase.

Streptavidin mixture supplied in the Detection kit is applied to the filtered material. This remains for 30 minutes at room temperature. The filters are then washed with PBS and the horseradish peroxidase is added to develop color. Peroxidase is maintained with citrate buffer. Color development occurs at 10 minutes. Filters must be read prior to 30 minutes of exposure.

Control nucleotide added should be visible in diluted strengths. Any nucleotide visible in antibody preparation should be quantitated and nucleotide contamination per dose established.

A reduction in nucleotide contamination with processing steps may be quantitated with this technique.

STERILITY: The antibody is sterile filtered through a 0.22 um filter prior to use. Filtered material, 0.1 ml, is placed in thioglycollate broth (GIBCO) and also onto chocolate agar plate (GIBCO) and incubated at 37°C (chocolate plate in $CO_2$ enriched atmosphere). The plate and tube are read at 24, 48, and 72 hours.

Filtered material is also inoculated onto Saboraud tubes (GIBCO) and incubated at room temperature and at 35°C for six weeks for fungal growth. The tubes are examined at 24 hours, 3 days, five days, and weekly.

Filtered material is also inoculated into a human embryo line (Bartel W1-38) and a monkey kidney line (Bartel RMK) to evaluate viral growth. A sample of the tumor line is also included for inoculation for evaluation of cytopathic effect. The lines are maintained at 35°C. The lines are examined at 7, 14, 21, 28 days for cytopathic effect. "O" red cells (Cooper Biological) are placed on the cells to evaluate adhesion.

Mycoplasma testing is also performed. Material is placed into a Mycotrim RS flask (Hana Biologicals) and the agar surface is scratched along the innoculated area. The flask is incubated at 35° with agar side up and observed daily for growth. At 72 hours the flask is reinnoculated and observed daily for growth for an additional 72 hours.

Limulus lysate testing is performed for endotoxin contamination (Sigma). 0.1 ml filtrate is injected into test vials and compared to simultaneously prepared vials containing E. coli endotoxin. At 1 hour of incubation at 37°C, filtrate is examined for hard gel formation.

All filtrates are examined for Hepatitis B virus and Human Immunodeficiency Virus (HIV) using ELISA (Abbott Laboratories). Filtrate is reacted with chromophore-linked antibody and the optical density is compared to that of known controls.

All tests are negative before filling the dose vial. Protein concentration is determined by method of Lowry (OD at 280nm).

EXAMPLE 2 -

The general procedures of Example 1 are repeated using human neurofibrosarcoma sensitized with adult spleen cells. The fusion line is composed of lymphoblast cells prepared according to Example 1. The resulting hybridoma (NFS-84B) secretes monoclonal idiotypic IgM antibodies which react with a 221,000 mw idiotypic surface antigen. The spleen line (AS-151) was deposited with the American Type Culture Collection (ATCC) on September 16, 1988 under ATCC No. CRL 9833. The lymphoblast fusion line (BM-95) was deposited with the ATCC on September 16, 1988 under ATCC No. CRL 9832. The hybridoma was deposited with the ATCC on September 16, 1988 under ATCC No. HB983. Antibody from this line was deposited with the FDA on October 23, 1987.

EXAMPLE 3 -

The general procedures of Example 1 are repeated using human neurofibrosarcoma sensitized with fetal spleen cells. The fusion line is composed of fetal marrow cells (BG-231) prepared according to Example 1 and deposited with the ATCC on September 16, 1988 under ATCC No. CRL 9835. The resulting hybridoma secretes monoclonal IgM antibodies which react with an idiotypic surface antigen.

EXAMPLE 4 -

The general procedures of Example 1 are repeated using human neurofibrosarcoma sensitized with adult spleen cells. The fusion line is composed of fetal marrow cells (BG-231) prepared according to Example 1. The resulting hybridoma secretes monoclonal IgG antibodies which react with an idiotypic surface antigen.

EXAMPLE 5 -

The general procedures of Example 1 are repeated using human neurofibrosarcoma sensitized with fetal spleen cells. The fusion line is composed of lymphoblastoid cells (BM-95) prepared according to Example 1 are deposited with the ATCC on September 16, 1988 under ATCC No. CRL 9832. The resulting hybridoma secretes monoclonal IgM antibodies which react with an idiotypic surface antigen.

EXAMPLE 6 -

The general procedures of Example 1 are repeated using human neurofibrosarcoma sensitized with adult spleen cells. The fusion line is composed of plasma cells (BA-160) prepared according to Example 1 and deposited with the ATCC on September 16, 1988 under ATCC No. CRL-9834. The resulting hybridoma secretes monoclonal IgA antibodies which react with an idiotypic surface antigen.

EXAMPLE 7 -

The general procedures of Example 1 are repeated using human neurofibrosarcoma sensitized with fetal spleen cells. The fusion line is composed of plasma cells (BA-160) prepared according to Example 1. The resulting hybridoma secretes monoclonal IgA antibodies which react with an idiotypic surface antigen.

EXAMPLE 8 -

The general procedures of Example 1 are repeated using adenocarcinoma cells of the lung sensitized with adult spleen cells. The fusion line is composed of lymphoblastoid cells prepared according to Example 1. The resulting hybridoma secretes monoclonal IgM antibodies which react with a idiotypic surface antigen.

EXAMPLE 9 -

The general procedures of Example 1 are repeated using adenocarcinoma cells of the lung sensitized with adult spleen cells. The fusion line is composed of plasma cells prepared according to Example 1. The resulting hybridoma secretes monoclonal IgA antibodies which react with idiotypic surface antigen.

EXAMPLE 10 -

The general procedures of Example 1 are repeated using adenocarcinoma cells of the breast sensitized with adult spleen cells. The fusion line is composed of lymphoblastoid cells prepared according to Example 1. The resulting hybridoma secretes monoclonal IgM antibodies which react with idiotypic surface antigen.

EXAMPLE 11 -

The general procedures of Example 1 are repeated using adenocarcinoma cells of the breast sensitized with adult spleen cells. The fusion line is composed of plasma cells prepared according to Example 1. The resulting hybridoma secretes monoclonal IgA antibodies which react with idiotypic surface antigen.

EXAMPLE 12 -

The general procedures of Example 1 are repeated using mucoepidermoil carcinoma cells sensitized with adult spleen cells. The fusion line is composed of lymphoblastoic cells prepared according to Example 1. The resulting hybridoma secretes monoclonal IgM antibodies which react with idiotypic surface antigen.

EXAMPLE 13 -

The general procedures of Example 1 are repeated using mucoepidermoil carcinoma cells sensitized with adult spleen cells. The fusion line is composed of plasma cells prepared according to Example 1. The resulting hybridoma secretes monoclonal IgA antibodies which react with idiotypic surface antigen.

EXAMPLE 14 -

The general procedures of Example 1 are repeated using hepatocellular carcinoma cells sensitized with adult spleen cells. The fusion line is composed of lymphoblastoid cells prepared according to Example 1. The resulting hybridoma secretes monoclonal IgM antibodies which react with idiotypic surface antigen.

EXAMPLE 15 -

The general procedures of Example 1 are repeated using hepatocellular carcinoma cells sensitized with adult spleen cells. The fusion line is composed of plasma cells prepared according to Example 1. The resulting hybridoma secretes monoclonal IgA antibodies which react with idiotypic surface antigen.

EXAMPLE 16 -

The general procedures of Example 1 are repeated using leiomyosarcoma cells sensitized with adult spleen cells. The fusion line is composed of lymphoblastoid cells prepared according to Example 1. The resulting hybridoma secretes IgM monoclonal antibodies which react with idiotypic surface antigen.

EXAMPLE 17 -

The general procedures of Example 1 are repeated using leiomyosarcoma cells sensitized with adult spleen cells. The fusion line is composed of plasma cells prepared according to Example 1. The resulting hybridoma secretes monoclonal IgA antibodies which react with idiotypic surface antigen.

EXAMPLE 18 -

The general procedures of Example 1 are repeated using melanoma cells sensitized with adult spleen cells. The fusion line is composed of lymphoblastoid cells prepared according to Example 1. The resulting hybridoma secretes monoclonal IgM antibodies which react with idiotypic surface antigen.

EXAMPLE 19 -

The general procedures of Example 1 are repeated using melanoma cells sensitized with adult spleen cells. The fusion line is composed of plasma cells prepared according to Example 1. The resulting hybridoma secretes monoclonal IgA antibodies which react with idiotypic surface antigen.

EXAMPLE 20 -

The general procedures of Example 1 are repeated using adenocarcinoma cells of the colon sensitized with adult spleen cells. The fusion line is composed of lymphoblastoid cells prepared according to Example 1. The resulting hybridoma secretes monoclonal IgM antibodies which react with idiotypic surface antigen.

EXAMPLE 21 -

The general procedures of Example 1 are repeated using adenocarcinoma cells of the colon sensitized with fetal spleen cells. The fusion line is composed of fetal marrow cells prepared according to Example 1. The resulting hybridoma secretes monoclonal IgG antibodies which react with idiotypic surface antigen.

EXAMPLE 22 -

The general procedures of Example 1 are repeated using adenocarcinoma cells of the colon sensitized with adult spleen cells. The fusion line is composed of fetal marrow cells prepared according to Example 1. The resulting hybridoma secretes monoclonal IgG antibodies which react with idiotypic surface antigen.

EXAMPLE 23 -

The general procedures of Example 1 are repeated using adenocarcinoma cells of the colon sensitized with fetal spleen cells. The fusion line is composed of lymphoblastoid cells prepared according to Example 1. The resulting hybridoma secretes monoclonal IgM antibodies which react with idiotypic surface antigen.

EXAMPLE 24 -

The general procedures of Example 1 are repeated using adenocarcinoma cells of the colon sensitized with adult spleen cells. The fusion line is composed of plasma cells prepared according to Example 1. The resulting hybridoma secretes monoclonal IgA antibodies which react with idiotypic surface antigen.

EXAMPLE 25 -

The general procedures of Example 1 are repeated using squamous carcinoma cells of the tongue sensitized with adult spleen cells. The fusion line is composed of lymphoblastoid cells prepared according to Example 1. The resulting hybridoma secretes monoclonal IgM antibodies which react with idiotypic surface antigen.

EXAMPLE 26 -

The general procedures of Example 1 are repeated using squamous carcinoma cells of the tongue sensitized with adult spleen cells. The fusion line is composed of plasma cells prepared according to Example 1. The resulting hybridoma secretes monoclonal IgA antibodies which react with idiotypic surface antigen.

EXAMPLE 27 -

The general procedures of Example 1 are repeated using adenocarcinoma cells of the pancreas sensitized with adult spleen cells. The fusion line is composed of lymphoblastoid cells prepared according to Example 1. The resulting hybridoma secretes monoclonal IgM antibodies which react with idiotypic surface antigen.

EXAMPLE 28 -

The general procedures of Example 1 are repeated using adenocarcinoma carcinoma cells of the pancreas sensitized with adult spleen cells. The fusion line is composed of plasma cells prepared according to Example 1. The resulting hybridoma secretes monoclonal IgA antibodies which react with idiotypic surface antigen.

EXAMPLE 29 -

The general procedures of Example 1 are repeated using acute leukemia lymphoblast cells sensitized with adult spleen cells. The fusion line is composed of lymphoblastoid cells prepared according to Example 1. The resulting hybridoma secretes monoclonal IgM antibodies which react with idiotypic surface antigen.

EXAMPLE 30 -

The general procedures of Example 1 are repeated using acute leukemia lymphoblast cells sensitized with adult spleen cells. The fusion line is composed of plasma cells prepared according to Example 1. The resulting hybridoma secretes monoclonal IgA antibodies which react with idiotypic surface antigen.

EXAMPLE 31 -

The general procedures of Example 1 are repeated using cells of Mycosis Fungoides sensitized with adult spleen cells. The fusion line is composed of lymphoblastoid cells prepared according to Example 1. The resulting hybridoma secretes monoclonal IgM antibodies which react with idiotypic surface antigen.

EXAMPLE 32 -

The general procedures of Example 1 are repeated using cells of Mycosis Fungoides sensitized with adult spleen cells. The fusion line is composed of plasma cells prepared according to Example 1. The resulting hybridoma secretes monoclonal IgA antibodies which react with idiotypic surface antigen.

EXAMPLE 33 -

The general procedures of Example 1 are repeated using oat cell carcinoma sensitized with adult spleen cells. The fusion line is composed of lymphoblastoid cells prepared according to Example 1. The resulting hybridoma secretes monoclonal IgM antibodies which react with idiotypic surface antigen.

EXAMPLE 34 -

The general procedures of Example 1 are repeated using oat cell carcinoma sensitized with adult spleen cells. The fusion line is composed of plasma cells prepared according to Example 1. The resulting hybridoma secretes monoclonal IgA antibodies which react with idiotypic surface antigen.

EXAMPLE 35 -

The general procedures of Example 1 are repeated using adenocarcinoma of the prostate gland sensitized with adult spleen cells. The fusion line is composed of lymphoblastoid cells prepared according to Example 1. The resulting hybridoma secretes monoclonal IgM antibodies which react with idiotypic surface antigen.

EXAMPLE 36 -

The general procedures of Example 1 are repeated using adenocarcinoma of the prostate gland sensitized with adult spleen cells. The fusion line is composed of plasma cells prepared according to Example 1. The resulting hybridoma secretes monoclonal IgA antibodies which react with idiotypic surface antigen.

EXAMPLE 37 -

The general procedures of Example 1 are repeated using squamous carcinoma of the esophagous sensitized with adult spleen cells. The fusion line is composed of lymphoblastoid cells prepared according to Example 1. The resulting hybridoma secretes monoclonal IgM antibodies which react with idiotypic surface antigen.

EXAMPLE 38 -

The general procedures of Example 1 are repeated using squamous carcinoma of the esophagous sensitized with adult spleen cells. The fusion line is composed of plasma cells prepared according to Example 1. The resulting hybridoma secretes monoclonal IgA antibodies which react with idiotypic surface antigen.

EXAMPLE 39 -

The general procedures of Example 1 are repeated using cells of Ewing's carcinoma sensitized with adult spleen cells. The fusion line is composed of lymphoblastoid cells prepared according to Example 1. The resulting hybridoma secretes monoclonal IgM antibodies which react with idiotypic surface antigen.

EXAMPLE 40 -

The general procedures of Example 1 are repeated using cells of Ewing's carcinoma sensitized with adult spleen cells. The fusion line is composed of plasma cells prepared according to Example 1. The resulting hybridoma secretes monoclonal IgA antibodies which react with idiotypic surface antigen.

EXAMPLE 41 -

The general procedures of Example 1 are repeated using adenocarcinoma of the stomach sensitized with adult spleen cells. The fusion line is composed of lymphoblastoid cells prepared according to Example 1. The resulting hybridoma secretes monoclonal IgM antibodies which react with idiotypic surface antigen.

EXAMPLE 42 -

The general procedures of Example 1 are repeated using adenocarcinoma of the stomach sensitized with adult spleen cells. The fusion line is composed of plasma cells prepared according to Example 1. The resulting hybridoma secretes monoclonal IgA antibodies which react with idiotypic surface antigen.

EXAMPLE 43 -

The general procedures of Example 1 are repeated using adenocarcinoma cells of the biliary tract sensitized with adult spleen cells. The fusion line is composed of lymphoblastoid cells prepared according to Example 1. The resulting hybridoma secretes monoclonal IgM antibodies which react with idiotypic surface antigen.

EXAMPLE 44 -

The general procedures of Example 1 are repeated using adenocarcinoma cells of the biliary tract sensitized with fetal spleen cells. The fusion line is composed of fetal marrow cells prepared according to Example 1. The resulting hybridoma secretes monoclonal IgG antibodies which react with idiotypic surface antigen.

EXAMPLE 45 -

The general procedures of Example 1 are repeated using adenocarcinoma cells of the biliary tract sensitized with adult spleen cells. The fusion line is composed of fetal marrow cells prepared according to Example 1. The resulting hybridoma secretes monoclonal IgG antibodies which react with idiotypic surface antigen.

EXAMPLE 46 -

The general procedures of Example 1 are repeated using adenocarcinoma cells of the biliary tract sensitized with fetal spleen cells. The fusion line is composed of lymphoblastoid cells prepared according to Example 1. The resulting hybridoma secretes monoclonal IgM antibodies which react with idiotypic surface antigen.

EXAMPLE 47 -

The general procedures of Example 1 are repeated using adenocarcinoma of the biliary tract sensitized with adult spleen cells. The fusion line is composed of plasma cells prepared according to Example 1. The resulting hybridoma secretes monoclonal IgA antibodies which react with idiotypic surface antigen.

EXAMPLE 48 -

The general procedures of Example 1 are repeated using mucinous adenocarcinoma of the ovary sensitized with adult spleen cells. The fusion line is composed of lymphoblastoid cells prepared according to Example 1. The resulting hybridoma secretes monoclonal IgM antibodies which react with idiotypic surface antigen.

EXAMPLE 49 -

The general procedures of Example 1 are repeated using mucinous adenocarcinoma of the ovary sensitized with adult spleen cells. The fusion line is composed of plasma cells prepared according to Example 1. The resulting hybridoma secretes monoclonal IgA antibodies which react with idiotypic surface antigen.

EXAMPLE 50 -

The general procedures of Example 1 are repeated using serous adenocarcinoma of the ovary sensitized with adult spleen cells. The fusion line is composed of lymphoblastoid cells prepared according to Example 1. The resulting hybridoma secretes monoclonal IgM antibodies which react with idiotypic surface antigen.

EXAMPLE 51 -

The general procedures of Example 1 are repeated using serous adenocarcinoma of the ovary sensitized with adult spleen cells. The fusion line is composed of plasma cells prepared according to Example 1. The resulting hybridoma secretes monoclonal IgA antibodies which react with idiotypic surface antigen.

EXAMPLE 52 -

The general procedures of Example 1 are repeated using lymphoblast cells from human lymphoma sensitized with adult spleen cells. The fusion line is composed of lymphoblastoid cells prepared according to Example 1. The resulting hybridoma secretes monoclonal IgM antibodies which react with idiotypic surface antigen.

EXAMPLE 53 -

The general procedures of Example 1 are repeated using lymphoblast cells from human lymphoma sensitized with adult spleen cells. The fusion line is composed of plasma cells prepared according to Example 1. The resulting hybridoma secretes monoclonal IgA antibodies which react with idiotypic surface antigen.

EXAMPLE 54 -

The general procedures of Example 1 are repeated using carcinoma of the alveolar cells sensitized with adult spleen cells. The fusion line is composed of lymphoblastoid cells prepared according to Example 1.

The resulting hybridoma secretes monoclonal IgM antibodies which react with idiotypic surface antigen.

EXAMPLE 55 -

The general procedures of Example 1 are repeated using carcinoma of the alveolar cells sensitized with adult spleen cells. The fusion line is composed of plasma cells prepared according to Example 1. The resulting hybridoma secretes monoclonal IgA antibodies which react with idiotypic surface antigen.

EXAMPLE 56 -

The general procedures of Example 1 are repeated using squamous carcinoma cells of the anus with adult spleen cells. The fusion line is composed of lymphoblastoid cells prepared according to Example 1. The resulting hybridoma secretes monoclonal IgM antibodies which reach with idiotypic surface antigen.

EXAMPLE 57 -

The general procedures of Example 1 are repeated using glioblastoma cells sensitized with adult spleen cells. The fusion line is composed of lymphoblastoid cells prepared according to Example 1. The resulting hybridoma secretes monoclonal IgM antibodies which react with idiotypic surface antigen.

**Claims**

1. A method for the production of human-human hybridomas comprising the steps of:
   (a) providing a suspension comprising cells from a human tumor and a suspension comprising human spleen cells;
   (b) sensitizing the human spleen cells in the suspension by mixing the cells with human tumor cells in the presence of a spleen cell stimulating agent;
   (c) fusing the human spleen cells and the human tumor cells with B-lymphocytes from a cell line distinct from the previously sensitized spleen cells;
   (d) screening the resultant fused cells to select at least one hybridoma that produces antibodies reactive with a specific human tumor antigen; and
   (e) cloning the selected hybridoma.

2. The method of claim 1, wherein said human spleen cells are obtained from a human fetus.

3. The method of claim 1 wherein said human spleen cells are obtained from adult human spleens and said suspension of adult human spleen cells are stimulated with (1) transfer factor, (2) a mitogen or (3) a lymphokine.

4. The method of claim 3, wherein the suspension of adult human spleen cells is stimulated with Phytohaemagglutinin (PHA) or Concanavalin A (Con A) or Interleukin-2 (IL2).

5. The method of claim 1, wherein the fusion line of B-lymphocytes is derived from a fetal marrow line, a human lymphoblastoid line or human plasma cells.

6. The method of claim 1, wherein single clones of a fusion line are selected which produce only one class of immunoglobulin.

7. The method of claim 1, wherein the human tumor cells are colon adenocarcinoma cells, lung adenocarcinoma cells, breast adenocarcinoma cells, mucoepidermoic carcinoma cells, hepatocellular carcinoma cells, leiomyosarcoma cells, melanoma cells, neuofibrosarcoma cells, tongue squamous carcinoma cells, pancreas adenocarcinoma cells, lymphoblast (acute leukemia) cells, Mycosis Fungoides cells, oat cell carcinoma cells, prostate adenocarcinoma cells, esophageal squamous carcinoma cells, Ewing's cells, gastric adenocarcinoma cells, biliary adenocarcinoma cells, ovary adenocarcinoma (mucinous) cells, ovary adenocarcinoma (serous) cells, lymphoblast (lymphoma) cells, alveolar cell carcinoma cells, squamous carcinoma cells of the anus or glioblastoma cells.

8. A method of producing monoclonal antibodies to each of the carcinoma cells according to claim 7, which comprises culturing a hybridoma according to claim 1 in a culture medium and recovering said antibodies from said medium.

9. A method for producing a continuous cell line that produces monoclonal antibodies to a specific cancer comprising the steps of:
   a) sensitizing human spleen cells in suspension by mixing the cells with a spleen cell stimulating agent in the presence of human tumor cells;

b) fusing the human spleen cells and the human tumor cells with human fetal marrow or lymphoblastic cells to produce hybridomas;

c) selecting from among said hybridomas a hybridoma that produces antibodies reactive with only one human tumor antigen; and

d) clonally expanding said selected hybridoma into a cell line.

## Patentansprüche

1. Verfahren zur Herstellung von Human-Human-Hybridomen, umfassend die Schritte des:

a) Bereitstellens einer Suspension, welche Zellen von einem humanen Tumor umfaßt, und einer Suspension, welche humane Milzzellen umfaßt;

(b) Sensibilisierens der humanen Milzzellen in der Suspension durch Mischen der Zellen mit humanen Tumorzellen in der Gegenwart eines Milzzellen-stimulierenden Agens;

(c) Fusionierens der humanen Milzzellen und der humanen Tumorzellen mit B-Lymphozyten von einer Zellinie, die von den zuvor sensibilisierten Milzzellen verschieden ist;

(d) Durchsuchens der resultierenden, fusionierten Zellen, um mindestens ein Hybridom zu selektieren, welches Antikörper produziert, die mit einem spezifischen, humanen Tumorantigen reaktionsfähig sind; und

(e) Klonierens des selektierten Hybridoms.

2. Verfahren nach Anspruch 1, worin die besagten humanen Milzzellen von einem humanen Fötus erhalten werden.

3. Verfahren nach Anspruch 1, worin die besagten humanen Milzzellen von adulten, humanen Milzen erhalten werden und die besagte Suspension adulter, humaner Milzzellen mit (1) Transfer-Faktor, (2) einem Mitogen oder (3) einem Lymphokin stimuliert werden.

4. Verfahren nach Anspruch 3, worin die Suspension adulter, humaner Milzzellen mit Phytohämagglutinin (PHA) oder Concanavalin A (Con A) oder Interleukin-2 (IL2) stimuliert wird.

5. Verfahren nach Anspruch 1, worin die Fusionslinie der B-Lymphozyten aus einer fötalen Knochenmarkslinie, einer humanen Lymphoblastoid-Linie oder humanen Plasmazellen abgeleitet wird.

6. Verfahren nach Anspruch 1, worin einzelne Klone einer Fusionslinie selektiert werden, welche nur eine Immunglobulin-Klasse produzieren.

7. Verfahren nach Anspruch 1, worin die humanen Tumorzellen Kolon-Adenokarzinomzellen, Lungen-Adenokarzinomzellen, Brust-Adenokarzinomzellen, Mucoepidermoid-Karzinomzellen, hepatozelluläre Karzinomzellen, Leiomyosarkomzellen, Melanomzellen, Neurofibrosarkomzellen, squamöse Zungen-Karzinomzellen, Pankreas-Adenokarzinomzellen, Lymphoblast (akute Leukämie)-Zellen, Mycosis-Fungoides-Zellen, Haferzell-Karzinomzellen, Prostata-Adenokarzinomzellen, squamöse Ösophagus-Karzinomzellen, Ewing-Zellen, Magen-Adenokarzinomzellen, Gallen-Adenokarzinomzellen, Eierstock-Adenokarzinomzellen (mucinartig), Eierstock-Adenokarzinomzellen (serös), Lymphoblast (Lymphom)-Zellen, Alveolarzellkarzinomzellen, squamöse Karzinomzellen des Anus oder Glioblastomzellen sind.

8. Verfahren zum Herstellen von monoklonalen Antikörpern gegen jede der Karzinomzellen nach Anspruch 7, welches das Kultivieren eines Hybridoms nach Anspruch 1 in einem Kulturmedium und das Gewinnen der besagten Antikörper von dem besagten Medium umfaßt.

9. Verfahren zum Herstellen einer kontinuierlichen Zellinie, welche monoklonale Antikörper gegen einen spezifischen Krebs produziert, umfassend die Schritte des:

(a) Sensibilisierens humaner Milzzellen in einer Suspension durch Mischen der Zellen mit einem Milzzellen-stimulierenden Agens in der Gegenwart humaner Tumorzellen;

(b) Fusionierens der humanen Milzzellen und der humanen Tumorzellen mit humanen, fötalen Knochenmarks- oder lymphoblastischen Zellen, um Hybridome zu produzieren;

(c) Selektierens aus den besagten Hybridomen ein Hybridom, welches Antikörper produziert, die mit nur einem humanen Tumorantigen reaktionsfähig sind; und

(d) klonalen Expandierens des besagten selektierten Hybridoms in eine Zellinie.

## Revendications

1. Procédé pour la production d'hybridomes humains-humains, comprenant les étapes suivantes:

(a) fourniture d'une suspension comprenant des cellules provenant d'une tumeur humaine et d'une suspension comprenant des cellules spléniques humaines;

(b) sensibilisation des cellules spléniques humaines dans la suspension par mélange des cellules avec les cellules tumorales en présence d'un agent stimulateur de cellules spléniques;

(c) fusion des cellules spléniques humaines et des cellules tumorales humaines avec des lymphocytes B provenant d'une lignée cellulaire distincte des cellules spléniques précédemment sensibilisées;

(d) criblage des cellules fusionnées résultantes pour sélectionner au moins un hybridome produisant des anticorps réactifs avec un antigène tumoral humain spécifique; et

(e) clonage des hybridomes sélectionnés.

2. Procédé selon la revendication 1, dans lequel lesdites cellules spléniques humaines sont obtenues à partir d'un foetus humain.

3. Procédé selon la revendication 1, dans lequel lesdites cellules spléniques humaines sont obtenues à partir de rates humaines d'adulte et les cellules spléniques humaines d'adulte dans ladite suspension sont stimulées par (1) un facteur de transfert, (2) un mitogène ou (3) une lymphokine.

4. Procédé selon la revendication 3, dans lequel les cellules spléniques humaines d'adulte dans la suspension sont stimulées par la phytohémagglutinine (PHA) ou la concanavaline (Con A) ou l'interleukine-2 (IL2).

5. Procédé selon la revendication 1, dans lequel la lignée de fusion de lymphocytes B provient d'une lignée médullaire foetale, d'une lignée de lymphoblastoïdes humaines ou de cellules plasmatiques humaines.

6. Procédé selon la revendication 1, dans lequel on sélectionne des clones individuels d'une lignée de fusion qui ne produisent qu'une seule classe d'immunoglobulines.

7. Procédé selon la revendication 1, dans lequel les cellules tumorales humaines sont des cellules d'adénocarcinome du côlon, des cellules d'adénocarcinome pulmonaire, des cellules d'adénocarcinome du sein, des cellules de carcinome mucoépidermoïde, des cellules d'hépatome, des cellules de leiomyosarcome, des cellules de mélanome, des cellules de neurofibrosarcome, des cellules d'épithélioma de la langue, des cellules d'adénocarcinome du pancréas, des cellules lymphoblastiques (de leucémie aiguë), des cellules de mycosis fongoïde, des cellules d'épithélioma à petites cellules du poumon, des cellules d'adénocarcinome de la prostate, des cellules d'épithélioma æsophagien, des cellules d'Ewing, des cellules d'adénocarcinome gastrique, des cellules d'adénocarcinome biliaire, des cellules d'épithélioma (colloïde) ovarien, des cellules (séreuses) d'épithélioma ovarien, des cellules lymphoblastiques (de lymphome), des cellules de carcinome bronchio-alvéolaire, des cellules d'épithélioma de l'anus ou des cellules de glioblastome.

8. Procédé de production d'anticorps monoclonaux dirigés contre des cellules de carcinome selon la revendication 7, comprenant la culture d'un hybridome selon la revendication 1, dans un milieu de culture, et la récupération desdits anticorps à partir dudit milieu.

9. Procédé pour la production d'une lignée cellulaire continue produisant des anticorps monoclonaux dirigés contre un cancer spécifique, comprenant les étapes suivantes:

a) sensibilisation de cellules spléniques humaines en suspension, par un agent stimulateur de cellules spléniques, en présence de cellules tumorales humaines;

b) fusion des cellules spléniques humaines et des cellules tumorales humaines avec des cellules médullaires foetales humaines ou des cellules lymphoblastiques humaines, pour la production d'hybridomes;

c) sélection parmi lesdits hybridomes d'un hybridome produisant des anticorps réactifs avec un seul antigène tumoral humain; et

d) amplification par clonage desdits hybridomes sélectionnés, pour l'obtention d'une lignée cellulaire.